(19) Europäisches Patentamt
European Patent Office
Office européen des brevets

(11) Veröffentlichungsnummer : **0 050 776**
**B1**

(12) **EUROPÄISCHE PATENTSCHRIFT**

(45) Veröffentlichungstag der Patentschrift : **16.11.83**

(51) Int. Cl.³ : **C 07 C 69/743**, C 07 C 69/74, C 07 C 67/317, C 07 C121/75

(21) Anmeldenummer : **81108190.0**

(22) Anmeldetag : **12.10.81**

(54) **Verfahren zur Herstellung von chlor-fluoralkenyl-substituierten Cyclopropancarbonsäureestern.**

(30) Priorität : **23.10.80 DE 3040001**
**21.11.80 DE 3043976**

(43) Veröffentlichungstag der Anmeldung : **05.05.82 Patentblatt 82/18**

(45) Bekanntmachung des Hinweises auf die Patenterteilung : **16.11.83 Patentblatt 83/46**

(84) Benannte Vertragsstaaten : **AT BE CH DE FR GB IT LI NL**

(56) Entgegenhaltungen :
**EP A 0 008 340**
**DE A 2 802 962**

(73) Patentinhaber : **BAYER AG**
**Zentralbereich Patente, Marken und Lizenzen**
**D-5090 Leverkusen 1, Bayerwerk (DE)**

(72) Erfinder : **Lantzsch, Reinhard, Dr.**
**Heymannstrasse 32**
**D-5090 Leverkusen (DE)**
Erfinder : **Hagemann, Hermann, Dr.**
**Kandinskystrasse 52**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 050 776 B1

Jouve, 18, rue St-Denis, 75001 Paris, France

Verfahren zur Herstellung von chlor-fluoralkenyl-substituierten Cyclopropancarbonsäureestern

Die Erfindung betrifft ein Verfahren zur Herstellung von chlor-fluoralkenyl-substituierten Cyclopropancarbonsäureestern durch Dehydrohalogenierung von chlorfluoralkyl-substituierten Cyclopropancarbonsäureestern.

Es ist bereits bekannt, 3-(2',2'-dichloro-3',3',3'-trifluoropropyl)-2,2-dimethyl-cyclopropancarbonsäureester durch Dehydrochlorierung in die entsprechenden (2'-Chloro-3',3',3'-trifluoropropenyl)-ester zu überführen, indem man in Anwesenheit eines Alkalicarbonats in einem polaren aprotischen Lösungsmittel arbeitet. Die dabei erreichbaren Ausbeuten von 55-63 % befriedigen jedoch für die technische Durchführung nicht (EP-A 10 859). Aus der nicht zum vorveröffentlichten Stand der Technik gehörenden EP-A 8340 ist 2,2-Dimethyl-3-(2'-chlor-3',3',3'-trifluorpropenyl)-cyclopropan carbonsäureethylester und seine Herstellung durch Dehydrohalogenierung von 3,3-Dimethyl-4,6,6-trichlor-7,7,7-trifluorönanthsäuremethylester bekannt.

Es wurde nun gefunden, daß man Ester der Formel I

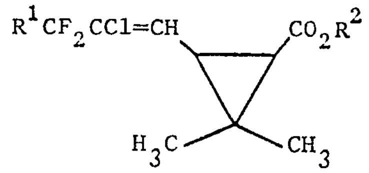

(I)

in welcher

$R^1$ für Fluor oder Trifluormethyl steht,

$R^2$ für niederes Alkyl oder für Pentafluorbenzyl, oder gegebenenfalls in α-Stellung CN-substituiertes 3-Phenoxy-4-fluor-benzyl

steht, erhält, indem man aus Estern der Formel II

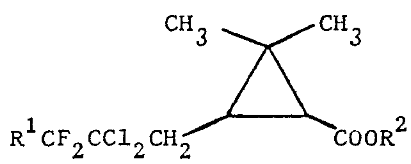

(II)

in welcher

$R^1$ und $R^2$ die oben angegebene Bedeutung haben, Chlorwasserstoff in einem polaren aprotischen Verdünnungsmittel abspaltet, dadurch gekennzeichnet, daß man entweder in Gegenwart von Alkalicarbonaten und quartären Ammonium- oder Phosphoniumsalzen arbeitet oder in Gegenwart von Kaliumfluorid arbeitet.

Diese Arbeitsweise führt zu Ausbeuten von 75-85 %, was eine beträchtliche und überraschende Steigerung gegenüber dem Stand der Technik darstellt.

Die Verbindungen der Formel II sind zum Teil bekannt, bzw. lassen sich analog zu bekannten Verfahren herstellen (vgl. DE-OS 2 907 609). Verbindungen der Formel II, in welcher $R^1$ für $CF_3$ steht, sind neu. An ihnen besteht besonderes Interesse, da sie zu hervorragenden Schädlingsbekämpfungsmitteln führen.

Bevorzugt wird das erfindungsgemäße Verfahren zur Herstellung von Verbindungen der Formel I durchgeführt, in welcher

$R^1$ für Fluor oder Trifluormethyl steht und

$R^2$ für $C_{1-4}$-Alkyl, Pentafluorbenzyl oder gegebenenfalls in α-Stellung CN-substituiertes 3-Phenoxybenzyl, das sowohl im Phenoxyteil als auch im Benzylteil substituiert sein kann,

steht.

Besonders bevorzugt werden Verbindungen hergestellt, in welchen

$R^2$ für Methyl, Ethyl, Pentafluorbenzyl oder 4-Fluor-3-phenoxybenzyl

steht.

Verwendet man beispielsweise 2,2-Dimethyl-3-(2,2-dichlor-3,3,4,4,4-pentafluor-butyl)-cyclopropancarbonsäurepentafluorbenzylester als Ausgangsstoff, so kann der Reaktionsverlauf durch das folgende Formelschema wiedergegeben werden :

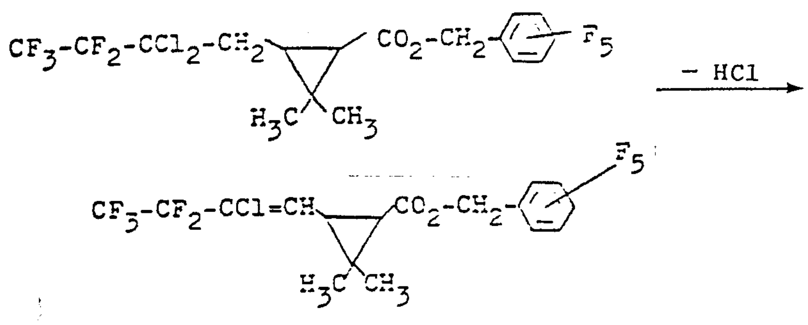

Die Chlorwasserstoffabspaltung aus den Verbindungen der Formel II erfolgt mit Hilfe eines Alkalicarbonats in Gegenwart eines polaren aprotischen Verdünnungsmittels sowie in Gegenwart eines als Katalysator dienenden quartären Ammonium- oder Phosphoniumsalzes, oder in Gegenwart von Kaliumfluorid in einem polaren aprotischen Verdünnungsmittel.

Als Verdünnungsmittel wird Dimethylformamid, Dimethylacetamid oder Acetonitril verwendet.

Als quartäre Ammoniumsalze werden bevorzugt eingesetzt : Tetrabutylammoniumbromid, Benzyltriethylammoniumchlorid oder Methyltrioctylammoniumchlorid.

Das Alkalicarbonat wird vorzugsweise äquimolar oder im Überschuß (bis 5-Molar) eingesetzt.

Besonders bevorzugt ist die Arbeitsweise in Acetonitril in Gegenwart von Kaliumcarbonat und einem quartären Ammoniumsalz.

Besonders bevorzugt ist aber auch die Arbeitsweise in Dimethylformamidin-Gegenwart von Kaliumfluorid.

## Beispiel 1

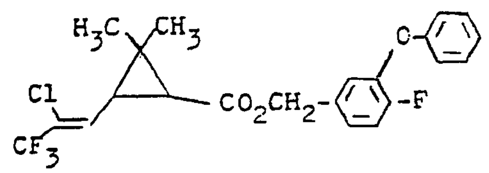

4 g cis/trans-2,2-Dimethyl-3-(2,2-dichlor-3,3,3-trifluorpropyl)-cyclopropancarbonsäure-4'-fluor-3'-phenoxy-benzylester werden in 20 ml Acetonitril gelöst. Man gibt 4 g Kaliumcarbonat und 0,5 g Tetrabutylammoniumbromid zu und erhitzt im Autoklaven 4 Stunden auf 180 °C. Nach dem Abkühlen wird mit Wasser verdünnt und mit Salzsäure neutral gestellt. Man extrahiert mit Methylenchlorid, trocknet mit Natriumsulfat, engt am Rotationsdampfer ein und destilliert bei 60 °C im Hochvakuum an Man erhält 3,4 g cis/trans-2,2-Dimethyl-3-(2-chlor-3,3,3-trifluorpropenyl)-cyclopropancarbonsäure-4'-fluor-3'-phenoxy-benzylester, wobei die Z-Isomeren ganz stark überwiegen.

Brechungsindex : $n_D^{20} = 1{,}519$.

## Beispiel 2

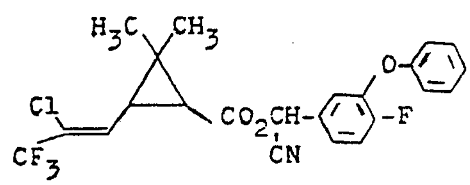

Analog Beispiel 1 wird aus 3,9 g cis/trans-2,2-Dimethyl-3-(2,2-dichlor-3,3,3-trifluorpropyl)-cyclopropancarbonsäure-4'-fluor-3'-phenoxy-α'-cyanobenzylester 3,5 g cis/trans-2,2-Dimethyl-3-(2-chlor-3,3,3-trifluorpropenyl)-cyclopropancarbonsäure-4'-fluor-3'-phenoxy-α'-cyano-benzylester erhalten.

## Beispiel 3

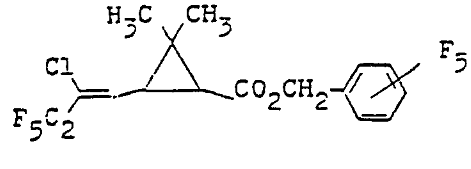

1,02 g (2 Mol) cis/trans-2,2-Dimethyl-3-(2,2-dichlor-3,3,4,4,4-pentafluor)-butyl-cyclopropancarbonsäurepentafluorbenzylester werden in 7 ml Dimethylacetamid gelöst und mit 0,6 g Kaliumfluorid versetzt und 4 Stunden auf 140 °C erhitzt. Nach dem Abkühlen wird mit Wasser verdünnt, dann sofort dreimal mit Ether extrahiert und die vereinigten Etherauszüge mit konz. Kochsalzlösung neutral gewaschen. Die organische Phase wird mit Natriumsulfat getrocknet, abfiltriert, einrotiert und im Hochvakuum andestilliert. Der Rückstand besteht aus cis/trans-2,2-Dimethyl-3-(2-chlor-3,3,4,4,4-pentafluoro-but-1-enyl)-cyclopropancarbonsäurepentafluorbenzylester.

Beispiel 4

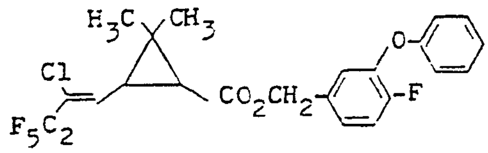

2 g cis/trans-2,2-Dimethyl-3-(2,2-dichlor-3,3,4,4,4-pentafluor)-butyl-cyclopropancarbonsäure-4'-fluor-3'-phenoxy-benzylester werden in 10 ml Acetonitril gelöst. Man gibt 1,5 g Kaliumcarbonat (feingepulvert) und 0,3 g Tetrabutylammoniumchlorid zu und erhitzt im Autoklaven 12 Stunden auf 170 °C. Die Aufarbeitung erfolgt analog Beispiel 1. Man erhält 1,6 g cis/trans-2,2-Dimethyl-3-(2-chlor-3,3,4,4,4-pentafluor-butenyl)-cyclopropancarbonsäure-4'-fluor-3'-phenoxy-benzylester.

Beispiel 5

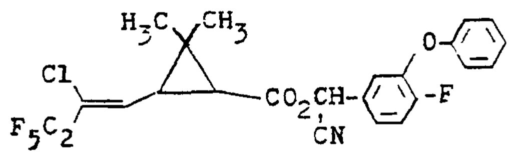

2,9 g cis/trans-2,2-Dimethyl-3-(2,2-dichlor-3,3,4,4,4-pentafluor)-butyl-cyclopropancarbonsäure-4'-fluor-3'-phenoxy-α'-cyano-benzylester werden in 10 ml Dimethylformamid gelöst. Man gibt 0,7 g gut getrocknetes Kaliumfluorid hinzu und erhitzt eine Stunde auf 150 °C. Nach dem Abkühlen wird mit Wasser verdünnt und neutral gestellt. Nach dreimaligem Extrahieren mit Ether werden die vereinigten Etherauszüge getrocknet und eingeengt. Der entstandene 2,2-Dimethyl-3-(2-chlor-3,3,4,4,4-pentafluor)-butenyl-cyclopropancarbonsäure-4'-fluor-3'-phenoxy-α'-cyano-benzylester wird chromatographisch (Kieselgel/n-Hexan/Chloroform 3 : 1) gereinigt.

Beispiel 6

307 g cis/trans-2,2-Dimethyl-3-(2,2-dichlor-3,3,3-trifluor-propyl)-cyclopropancarbonsäureethylester werden in 1 000 ml Acetonitril gelöst. Man gibt 140 g Kaliumcarbonat und 20 g Methyltrioctylammoniumchlorid zu und erhitzt im Autoklaven 8 Stunden auf 180 °C. Nach dem Abkühlen wird mit Wasser verdünnt, mit Methylenchlorid extrahiert und mit verdünnter Salzsäure und dann mit Wasser neutral gewaschen. Nach Trocknen mit Natriumsulfat wird einrotiert. Durch fraktionierte Destillation erhält man 230,5 g (85 % d. Th.) 2,2-Dimethyl-3-(2-chlor-3,3,3-trifluorpropenyl)-cyclopropancarbonsäureethylester vom Siedepunkt Kp = 94-96°/18 mbar.

**Ansprüche**

1. Verfahren zur Herstellung von Estern der Formel I

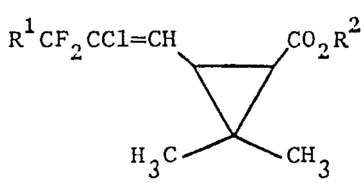

(I)

in welcher

$R^1$ für Fluor oder Trifluormethyl steht,

$R^2$ für niederes Alkyl oder für Pentafluorbenzyl oder gegebenenfalls in α-Stellung CN-substituiertes 3-Phenoxy-4-fluorbenzyl

steht, indem man aus Estern der Formel II

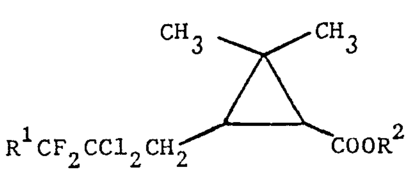

(II)

in welcher

$R^1$ und $R^2$ die oben angegebene Bedeutung haben, Chlorwasserstoff in einem polaren aprotischen Verdünnungsmittel abspaltet, dadurch gekennzeichnet, daß man entweder in Gegenwart von Alkalicarbonaten und quartären Ammonium- oder Phosphoniumsalzen arbeitet oder in Gegenwart von Kaliumfluorid arbeitet.

2. Ester der Formel II

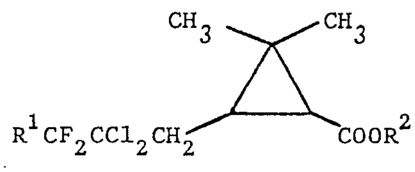

(II)

in welcher

$R^1$ die in Anspruch 1 angegebene Bedeutung hat, und

$R^2$ für Pentafluorbenzyl, gegebenenfalls in α-Stellung CN-substituiertes 3-Phenoxy-4-fluor-benzyl, steht.

**Claims**

1. Process for the production of esters of the formula I

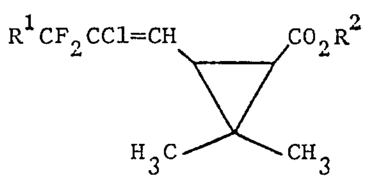

(I)

in which

$R^1$ represents fluorine or trifluoromethyl,

$R^2$ represents lower alkyl or pentafluorobenzyl or 3-phenoxy-4-fluorobenzyl optionally substituted by CN in the α-position,

by splitting off hydrogen chloride from esters of the formula II

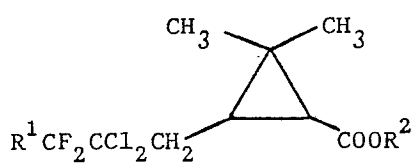

(II)

in which

$R^1$ and $R^2$ have the meaning indicated above, in a polar aprotic diluent, characterised in that either the reaction is carried out in the presence of alkali metal carbonates and quaternary ammonium or phosphonium salts or the reaction is carried out in the presence of potassium fluoride.

2. Esters of the formula II

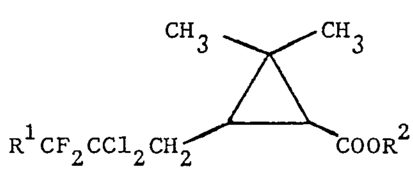

(II)

in which

$R^1$ has the meaning indicated in Claim 1 and

$R^2$ represents pentafluorobenzyl or 3-phenoxy-4-fluorobenzyl which is optionally substituted by CN in the α-position.

**Revendications**

1. Procédé de préparation d'esters de formule I

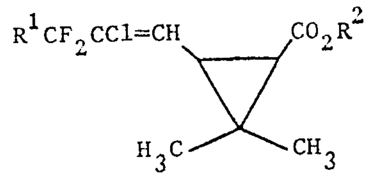

(I)

dans laquelle

$R^1$ représente un atome de fluor ou un groupe trifluorométhyle,

$R^2$ représente un groupe alkyle inférieur ou un groupe pentafluorobenzyle ou encore un groupe 3-phénoxy-4-fluorobenzyle éventuellement substitué en position α par un groupe CN,
en séparant du chlorure d'hydrogène d'esters de formule II

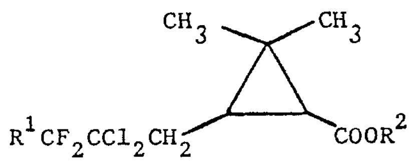

(II)

dans laquelle

$R^1$ et $R^2$ ont les significations indiquées ci-dessus, dans un diluant aprotique polaire, caractérisé en ce qu'on travaille en présence de carbonates alcalins et de sels de phosphonium ou d'ammonium quaternaire ou en présence de fluorure de potassium.

2. Esters de formule II

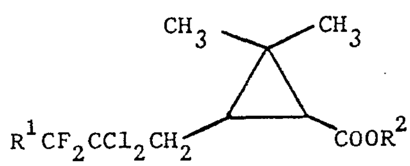

(II)

dans laquelle

$R^1$ a la signification indiquée dans la revendication 1, et

$R^2$ représente un groupe pentafluorobenzyle ou un groupe 3-phénoxy-4-fluoro-benzyle éventuellement substitué en position α par un groupe CN.